# EUROPEAN PATENT SPECIFICATION

(11) **EP 2 578 194 B1**
(45) Date of publication and mention of the grant of the patent: **21.11.2018**
(21) Application number: 11789794.2
(22) Date of filing: 31.05.2011
(51) Int. Cl.: A61F 13/15, A61F 13/539, A61F 13/56

(54) **ABSORBENT ARTICLE**
SAUGFÄHIGER ARTIKEL
ARTICLE ABSORBANT

(30) Priority: 31.05.2010 JP 2010125215
(43) Date of publication of application: 10.04.2013
(73) Proprietor: Unicharm Corporation, Ehime 799-0111 (JP)
(72) Inventor: MINAMI, Mari, Kanonji-shi Kagawa 769-1602 (JP); KUDO, Jun, Kanonji-shi Kagawa 769-1602 (JP); KINOSHITA, Hideyuki, Kanonji-shi Kagawa 769-1602 (JP); TAKAHASHI, Yuji, Kanonji-shi Kagawa 769-1602 (JP)
(74) Representative: Staeger & Sperling Partnerschaftsgesellschaft mbB
(86) International application number: PCT/JP2011/062471
(87) International publication number: WO 2011/152391

(56) References cited:
- WO-A1-2009/139248
- WO-A1-2012/029314
- JP-A- 2004 181 085
- JP-A- 2005 007 075
- JP-A- 2005 177 087
- JP-A- 2007 105 198
- JP-A- 2007 195 665
- JP-A- 2007 195 665
- JP-A- 2007 275 491
- JP-A- 2007 275 491
- JP-A- 2008 500 107
- US-A1- 2004 243 087
- US-A1- 2006 276 767

## Description

### [TECHNICAL FIELD]

The present invention relates to an absorbent article.

### [BACKGROUND ART]

Conventionally, there is known an absorbent article provided with a compressed portion configured to have compression from a topsheet to an absorber (for example, see Patent Literature 1).

Such a compressed portion increases a rigidity of the absorbent article, so that a twist or misalignment can be prevented from occurring even in a case where the absorbent article is worn for a long time.

### [CITATION LIST]

### [PATENT LITERATURE]

Patent Literature 1: Japanese Patent Application Publication No. 2007-7456. Further prior art in this technical field is disclosed in documents WO 2012/029314 A1, WO 2009/139248, JP 2007 195665 A, US 2006/276767 A1, US 2004/243087 A1, JP 2007 275491 A.

### [SUMMARY OF INVENTION]

However, the applicants faced the following problem as regard the above absorbent article.

In the absorbent article such as shown in Fig. 1 of Patent Literature 1, because the aforementioned compressed portion is provided without being divided in a longitudinal direction of the absorbent article, the absorbent article cannot get deformed flexibly according to irregularity of a wearer's body, thereby leading to a problem that the fitting into the wearer's body is diminished.

Further, in the absorbent article such as shown in Fig. 10 of Patent Literature 1, a gap part is provided in the longitudinal direction between divided pieces of the compressed portion, thereby leading to a problem that this gap part is possibly twisted to cause leakage of bodily fluid.

Thus, the present invention has been achieved in view of the aforementioned problem, and an object thereof is to provide an absorbent article which is capable of getting deformed flexibly according to irregularity of a wearer's body.

A first feature of the present invention is summarized as an absorbent article according to claims 1-7.

### [BRIEF DESCRIPTION OF DRAWINGS]

[Fig. 1] Fig. 1 is a plan view of an absorbent article according to the present invention, as seen from a skin contact surface side.
[Fig. 2] Fig. 2 shows a cross-sectional view taken along a line a-a and a cross-sectional view taken along a line b-b in the plan view of the absorbent article according to the present invention, as seen from the skin contact surface side.
[Fig. 3] Fig. 3 is a plan view of an absorbent article not part of the present invention, as seen from a clothing contact surface side.
[Fig. 4] Fig. 4 is a view for explaining a method of manufacturing the absorbent article according to the present invention.
[Fig. 5] Fig. 5 is a view for explaining a state in which the absorbent article according to the present invention gets deformed when it is worn.
[Fig. 6] Fig. 6 is a plan view of an absorbent article according to the present invention, as seen from a skin contact surface side.

### [DESCRIPTION OF EMBODIMENTS]

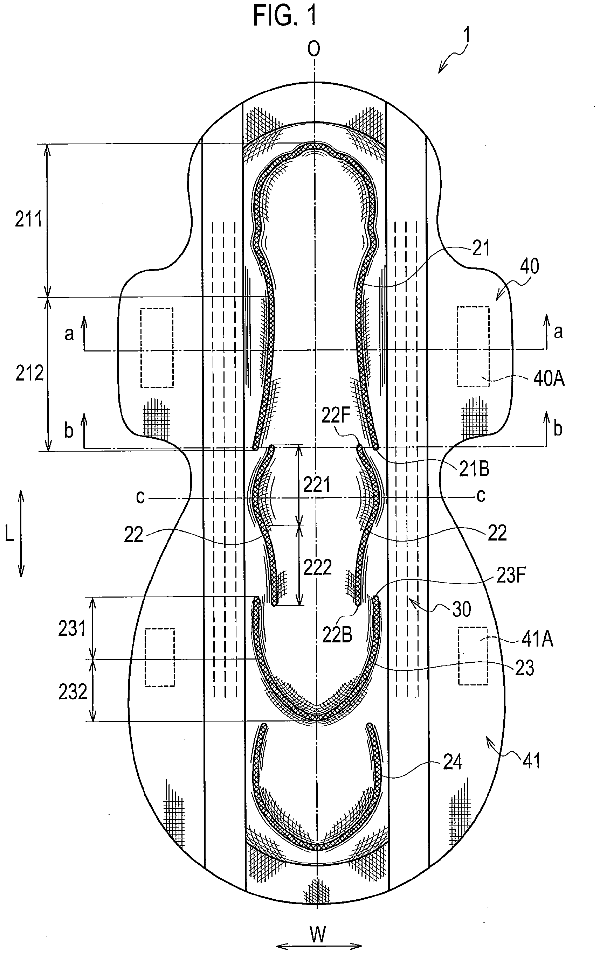

With reference to Fig. 1 to Fig. 5, an absorbent article 1 according to the present invention will be described. For example, the absorbent article 1 according to the present embodiment is a paper diaper, an incontinence pad, sanitary napkin, or the like.

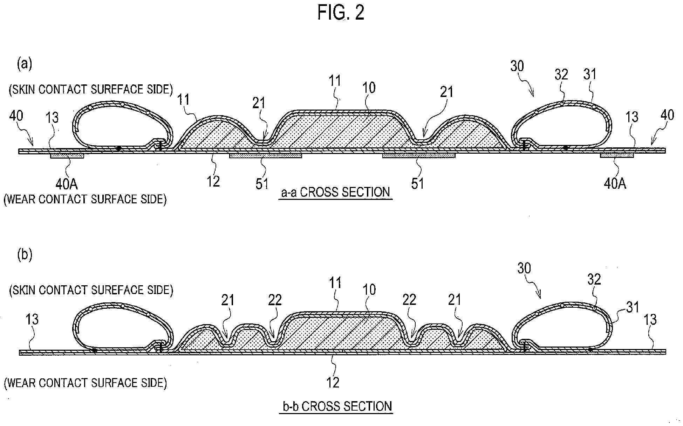

As shown in Fig. 1, Fig, 2(a), and Fig. 2(b), the absorbent article 1 according to the present embodiment is provided with a compressed portion configured to have compression from a topsheet 11 to an absorber 10 in a longitudinal direction L.

As shown in Fig. 1, this compressed portion is divided into a central compressed portion 21, a first rear compressed portion 22, a second rear compressed portion 23, and a third rear compressed portion 24.

The central compressed portion 21 is provided so as to cross a central line (a-a) of an excretion portion contact portion of the absorbent article 1, in the longitudinal direction L.

On the other hand, in the longitudinal direction L, the first rear compressed portion 22, the second rear compressed portion 23, and the third rear compressed portion 24 are provided posteriorly from the central line (a-a) of the excretion portion contact portion.

Note that the excretion portion contact portion of the absorbent article 1 according to the present embodiment is a portion which comes in contact with a vaginal opening of a wearer, in the absorbent article 1. For example, it is a portion which is disposed between two leg hole openings of underwear when the absorbent article 1 is worn on the underwear of the wearer.

Further, the absorbent article 1 according to the present embodiment may have a pair of central wing portions 40, which are formed so as to protrude outwardly in a width direction W at the excretion portion contact portion. Further, the absorbent article 1 according to the present embodiment may have a pair of rear wing portions 41 posteriorly from the central wing portions 40 in the longitudinal direction L.

Herein, when wearing the absorbent article 1 on the underwear, the wearer fixes the absorbent article 1 to the underwear by wrapping the central wing portions 40 around a crotch of the underwear and spreading the rear wing portions 41 on the underwear.

Therefore, for example, in a case where the central wing portions 40 are provided in the absorbent article 1 according to the present embodiment, the central line (a-a) of the aforementioned excretion portion contact portion corresponds to a central line of the central wing portions 40 in the longitudinal direction L.

Note that, in a case where the central wing portions 40 are not provided, when wearing the absorbent article 1 on the underwear, the wearer fixes the absorbent article 1 on the underwear by adjusting a position having the shortest length in the width direction W of the absorbent article 1, to the crotch of the underwear.

Therefore, for example, in a case where the central wing portions 40 are not provided in the absorbent article 1 according to the present embodiment, the central line (a-a) of the aforementioned excretion portion contact portion is a line which extends in the width direction W at a position having the shortest length in the width direction W of the absorbent article 1.

Further, in the longitudinal direction L, a rear end 21B of the central compressed portion 21 is provided posteriorly from a front end 22F of the first rear compressed portion 22.

Yet further, in the width direction W, the rear end 21B of the central compressed portion 21 is provided outwardly from the front end 22F of the first rear compressed portion 22.

Herein, the central compressed portion 21 may be formed so as to expand outwardly in the width direction W as it extends toward the rear end 21B at a rear portion 212 in the longitudinal direction L, or may be formed so as not to expand outwardly in the width direction W.

On the other hand, the central compressed portion 21 may be divided at a front portion 211 in the longitudinal direction L, or may be continuous without division.

Further, the first rear compressed portion 22 may be formed so as to narrow inwardly in the width direction W as it extends toward the front end 22F at a front portion 221 in the longitudinal direction L, or may be formed so as not to narrow inwardly in the width direction W.

Yet further, in the longitudinal direction L, a rear end 22B of the first rear compressed portion 22 may be provided posteriorly from a front end 23F of the second rear compressed portion 23.

In addition, in the width direction W, the rear end 22B of the first rear compressed portion 22 may be provided inwardly from the front end 23F of the second rear compressed portion 23.

Herein, the first rear compressed portion 22 may be formed so as to narrow inwardly in the width direction W as it extends toward the rear end 22B at a rear portion 222 in the longitudinal direction L, or may be formed so as not to narrow inwardly in the width direction W.

Note that the second rear compressed portion 23 may be formed so as to expand outwardly in the width direction W as it extends toward the front end 23F at a front portion 231 in the longitudinal direction L, or may be formed so as not to expand outwardly in the width direction W.

On the other hand, the second rear compressed portion 23 may be divided at a rear portion 232 in the longitudinal direction L, or may be continuous without division.

As shown in Fig. 2(a) and Fig. 2(b), the absorbent article 1 according to the present embodiment has a liquid-permeable topsheet 11, a liquid-impermeable backsheet 12, sidesheets 13 configuring the central wing portions 40, and an absorber 10 disposed between the topsheet 11 and the backsheet 12. Note that the absorbent article 1 according to the present embodiment may have a second sheet between the topsheet 11 and the absorber 10.

The absorber 10 is configured by ground pulp, a superabsorbent polymer, and a core wrap for wrapping both. Herein, a macromolecular polymer is a granular polymer of a sodium acrylate copolymer.

Further, the core wrap is a sheet made from an arbitrary material which absorbs liquid. For example, as the core wrap, a tissue may be used, or an air-laid sheet formed by shaping a hydrophilic fiber or ground pulp into a sheet by an air-laid method may be used.

Yet further, the absorbent article 1 according to the present embodiment may be provided with a leakage-preventing wall 30 in the longitudinal direction L. This leakage-preventing wall 30 is configured by a leakage-preventing sheet 31 folded up in midair and an elastic member 32 disposed in a stretched state inside the leakage preventing sheet 31.

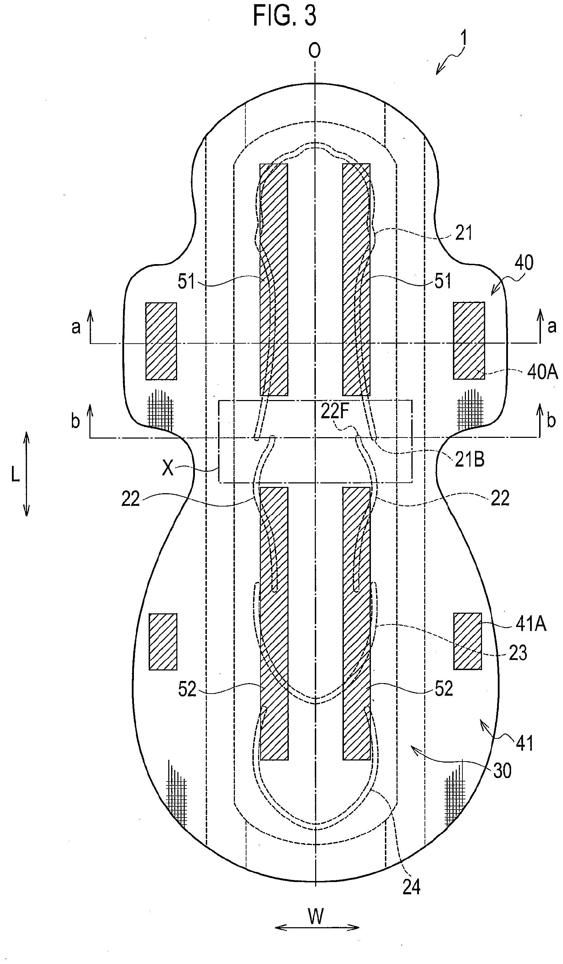

As shown in Fig. 3 (not part of the present invention), in the absorbent article 1, a clothing contact surface side of the backsheet is coated with an adhesive for adhering the clothing contact surface of the backsheet 12 to an inner surface of underwear.

For example, such an adhesive is coated on portions 40A inside the central wing portions 40 and portions 41A inside the rear wing portions 41.

Note that such an adhesive is not coated on an overlapping portion X but coated on a portion other than the overlapping portion X at the clothing contact surface side of the backsheet 12.

Herein, as shown in Fig. 3 (not part of the present invention), at the clothing contact surface side of the backsheet 12, the overlapping portion X is a portion which extends in the width direction W from one side edge to the other side edge of the absorbent article 1, and which corresponds to a portion including the rear end 21B of the central compressed portion 21 and the front end 22F of the rear compressed portion 22.

With such a configuration, the absorbent article 1 and the underwear are not adhered to each other at the overlapping portion X, thereby making it easy to fit the absorbent article 1 into irregularity of the wearer's body without the influence of movement of the underwear in association with, e.g., turning over of the wearer.

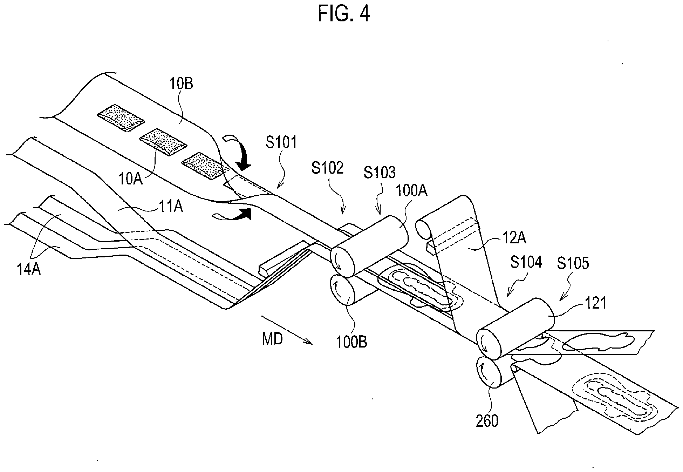

Next, some of the methods of manufacturing the absorbent article 1 according to the embodiment are described with reference to Fig. 4. Note that as far as the methods that are not described in Fig. 4 are concerned, the existing methods can be used.

As shown in Fig. 4, in step S101, the absorber 10 is generated by wrapping a core wrap 10B around a laminated body 10A of the ground pulp and the absorbent polymer.

In step S102, the absorber 10 is arranged on a web 11A for the topsheet 11 which is conveyed in continuation, and the web 11A for the topsheet 11 and the absorber 10 are bonded to each other using an adhesive.

In step S103, the web 11A for the topsheet 11 and the absorber 10, which are bonded together, are subjected to a compressing process using a press rollers 100A, 100B, thereby forming the central compressed portion 21, the first rear compressed portion 22, the second rear compressed portion 23, and the third rear compressed portion 24.

In step S104, a web 12A for the backsheet 12 is bonded to the web 11A for the topsheet 11 and the absorber 10 to which the compressing processing has been performed.

In step S105, the web 11A for the topsheet 11, the absorber 10, and the backsheet 12A for the backsheet 12, all of which have been bonded together, are cut in a predetermined size and shape using a product cutter, thereby generating the absorbent article 1.

According to the absorbent article 1 of the present embodiment, because the central compressed portion 21 and the rear compressed portions 22 to 24 are divided so that force transmitted from the crotch portion of the wearer to the central compressed portion 21 is not transmitted directly to the rear compressed portions 22 to 24, the absorbent article 1 is capable of getting deformed flexibly according to the irregularity of the wearer's body.

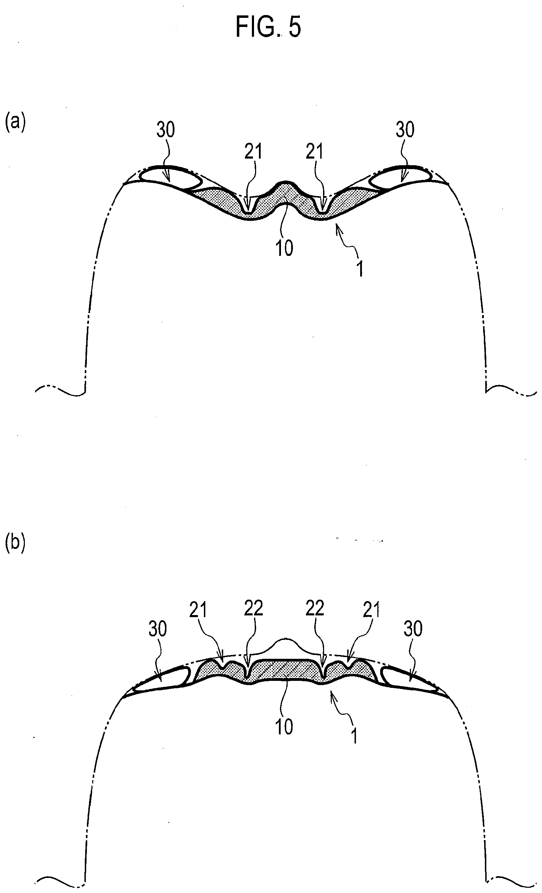

Specifically, at a neighboring portion of the central line (a-a) of the excretion portion contact portion, that is, at a portion which comes in contact with the crotch portion of the wearer, as shown in Fig. 5(a), the side edge of the absorber 10 is configured to rise along the central compressed portion 21 by force from the crotch portion of the wearer, so that the absorbent article 1 can be fit into the irregularity of the wearer's body without any gaps.

On the other hand, at the rear side in the longitudinal direction L of the central line (a-a) of the excretion portion contact portion (for example, a line (c-c) in the width direction shown in Fig. 1), that is, at a portion which comes in contact with the buttocks of the wearer, as shown in Fig. 5(b), the force from the crotch portion of the wearer is transmitted indirectly to the rear compressed portions 22 to 24 through the central compressed portion 21, so that the side edge of the absorber 10 does not rise and the absorbent article 1 can be fit into the irregularity of the wearer's body without any gaps.

According to the absorbent article 1 of the present embodiment, in the longitudinal direction L, the rear end 21B of the central compressed portion 21 is provided posteriorly from the front end 22F of the first rear compressed portion 22, so that the bodily fluid flowing in the width direction W can be absorbed by both of the central compressed portion 21 and the first rear compressed portion 22, thereby being able to prevent leakage of the bodily fluid in the width direction W.

According to the absorbent article 1 of the present embodiment, in the longitudinal direction L, the rear end 21B of the central compressed portion 21 is provided posteriorly from the front end 22F of the first rear compressed portion 22, so that in the longitudinal direction L, a gap part does not exist between the central compressed portion 21 and the first rear compressed portion 22, thereby being able to prevent leakage of the bodily fluid due to a twist of such a gap part.

According to the absorbent article 1 of the present embodiment, in a case where the central compressed portion 21 is formed so as to expand outwardly in the width direction W as it extends toward the rear end 21B at the rear portion 212 in the longitudinal direction L, at the central compressed portion 21, the force from the crotch portion of the wearer is guided outwardly in the width direction W, thereby further ensuring that the force is not transmitted to the rear compressed portions 22 to 24.

According to the absorbent article 1 of the present embodiment, in a case where the rear compressed portion 22 is formed so as to narrow inwardly in the width direction W as it extends toward the front end 22F at the front portion 221 in the longitudinal direction L, it is more difficult to transmit the force from the crotch portion of the wearer through the central compressed portion 21 to the rear compressed portion 22.

According to the absorbent article 1 of the present embodiment, the first rear compressed portion 22 narrows inwardly in the width direction W as it extends toward the rear end 22B at the rear portion 222 in the longitudinal direction L, so that the absorbent article 1 gets easily deformed in the longitudinal direction L of the absorbent article 1 while being inhibited from getting deformed in the width direction W, thereby making it easy to fit the absorbent article 1 into a curve of the wearer's buttocks.

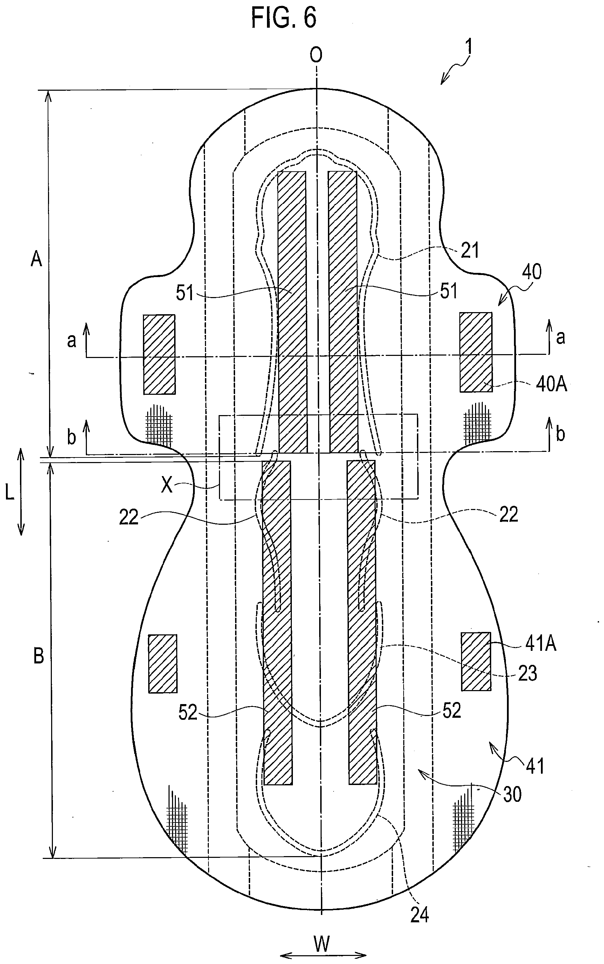

With reference to Fig. 6, the absorbent article 1 according to the present invention will be described.

As shown in Fig. 6, in the absorbent article 1 according to the present invention, at a central portion A, an adhesive for adhering the clothing contact surface of the backsheet 12 to the inner surface of the underwear is not coated on a portion corresponding to an outward in the width direction W of the central compressed portion 21 but coated on a portion corresponding to an inward in the width direction W of the central compressed portion 21.

Herein, at the central portion A, as long as such an adhesive is coated on at least part of the portion corresponding to the inward in the width direction W of the central compressed portion 21, the adhesive may be coated on, for example, a portion in an substantially rectangular shape within the portion corresponding to the inward in the width direction W of the central compressed portion 21, as shown in Fig. 6.

Note that at the central portion A, the adhesive may be coated on at least part of a portion corresponding to the central compressed portion 21.

On the other hand, as shown in Fig. 6, in the absorbent article 1 according to the present invention, at a rear portion B, the adhesive is not coated on a neighboring portion of a central line O in the width direction but coated on an outward in the width direction W of the neighboring portion of the central line O in the width direction W.

Herein, at the rear portion B, as long as such an adhesive is coated outwardly in the width direction W from the neighboring portion of the central line O in the width direction W, the adhesive portion may be coated on any of a portion corresponding to the inward in the width direction W of the first rear compressed portion 22, a portion corresponding to the outward in the width direction W of the first rear compressed portion 22, and a portion corresponding to the first rear compressed portion 22.

For example, as shown in Fig. 6, at the rear portion B, the adhesive may be coated across the portion corresponding to the inward in the width direction W of the first rear compressed portion 22, the portion corresponding to the outward in the width direction W of the first rear compressed portion 22, and the portion corresponding to the first rear compressed portion 22.

Further, as shown in Fig. 6, a coating position of the adhesive at the rear portion B may be provided outwardly in the width direction W from a coating position of the adhesive at the central portion A.

Herein, the central portion A is a portion corresponding to a portion provided with the central compressed portion 21 at the clothing contact surface side of the backsheet 12, and the rear portion B is a portion provided outwardly in the longitudinal direction L from the central portion A.

According to the absorbent article 1 of the present invention, it becomes easy for the side edge of the absorber 10 to rise at the central portion A and for the neighboring portion of the central line O in the width direction W to get deformed at the rear portion B, so that the absorbent article 1 can be fit into the irregularity of the wearer's body without any gaps.

### (Modification)

The absorbent article 1 according to the invention described above may be configured so that the leakage-preventing wall 30 is provided within a portion (for example, the portion corresponding to the central portion A in Fig. 6) provided with the central compressed portion 21 in the longitudinal direction L.

With such a configuration, contraction of the leakage-preventing wall 30 can be completed within the portion provided with the central compressed portion 21, thereby being able to reduce the linkage between the central compressed portion 21 and the first rear portion.

### [INDUSTRIAL APPLICABILITY]

As described above, it is possible to provide the absorbent article which is capable of getting deformed flexibly according to irregularity of a wearer's body.

### [Reference Signs List]

1..,Absorbent article
10...Absorber
11...Topsheet
12...Backsheet
13...Sidesheet
21...Central compressed portion
22...First rear compressed portion
23...Second rear compressed portion
24...Third rear compressed portion
30...Leakage-preventing wall
31...Leakage-preventing sheet
40...Central wing portion
41...Rear wing portion

## Claims

1. An absorbent article (1), comprising:
a topsheet (11) of liquid-permeable;
a backsheet (12) of liquid-impermeable; and
an absorber (10) disposed between the topsheet (11) and the backsheet (12), wherein
a compressed portion configured to have compression from the topsheet (11) to the absorber (10) is provided in a longitudinal direction of the absorbent article (1),
the compressed portion is divided into at least a central compressed portion (21) and a rear compressed portion,
the central compressed portion (21) is provided in the longitudinal direction so as to cross a central line (a-a) of an excretion portion contact portion of the absorbent article (1),
the rear compressed portion is provided posteriorly in the longitudinal direction (L) from the central line (a-a) of the excretion portion contact portion,
a rear end (21B) of the central compressed portion (21) is provided posteriorly in the longitudinal direction (L) from a front end (22F) of the rear compressed portion, and
the rear end (21B) of the central compressed portion (21) is provided outwardly in a width direction (W) of the absorbent article (1) from the front end (22F) of the rear compressed portion, **characterised in that**
a clothing contact surface side of the backsheet (12) is provided with a central portion (A) corresponding to a portion provided with the central compressed portion (21), and a rear portion (B) provided posteriorly in the longitudinal direction (L) from the central portion (A),
at the central portion (A), an adhesive for adhering a clothing contact surface of the backsheet (12) to an inner surface of underwear is not coated on a portion corresponding to an outward in the width direction (W) of the central compressed portion (21) but coated on a portion corresponding to an inward in the width direction of the central compressed portion (21), and
at the rear portion (B), the adhesive is not coated on a neighboring portion of a central line (O) in the width direction (W) but coated on an outward in the width direction (W) of the neighboring portion of the central line (O) in the width direction (W).

2. The absorbent article according to claim 1, wherein the central compressed portion (21) is formed so as to expand outwardly in the width direction (W) as it extends toward the rear end at the rear portion (B) in the longitudinal direction.

3. The absorbent article according to claim 1 or 2, wherein the rear compressed portion is formed so as to narrow inwardly in the width direction (W) as it extends toward the front end at a front portion in the longitudinal direction (L).

4. The absorbent article according to any one of claims 1 to 3, wherein
the rear compressed portion is divided into at least a first rear compressed portion (22) and a second rear compressed portion (23), and
a rear end of the first rear compressed portion (22) is provided posteriorly in the longitudinal direction and inwardly in the width direction with respect to a front end of the second rear compressed portion (23).

5. The absorbent article according to any one of claims 1 to 4, wherein
a clothing contact surface side of the backsheet (12) is provided with an overlapping portion as a portion extending in the width direction from one side edge to the other side edge of the absorbent article (1) as well as a portion corresponding to a portion including the rear end of the central compressed portion (21) and the front end of the rear compressed portion, and
an adhesive for adhering a clothing contact surface of the backsheet (12) to an inner surface of underwear is not coated on the overlapping portion but coated on a portion other than the overlapping portion at the clothing contact surface side of the backsheet (12).

6. The absorbent article according to any one of claims 1 to 5, wherein
central wing portions (40) configured to protrude outwardly at both sides in the width direction are provided, and
the central line of the excretion contact portion corresponds to a central line of the central wing portions (40) in the longitudinal direction.

7. The absorbent article according to any one of claims 1 to 5, wherein
central wing portions (40) configured to protrude outwardly at both sides in the width direction are not provided, and
the central line of the excretion contact portion is a line which extends in the width direction at a position having the shortest length in the width direction of the absorbent article (1).

## Patentansprüche

1. Ein absorbierender Artikel (1), umfassend:
eine flüssigkeitsdurchlässige Oberlage (11);
eine flüssigkeitsundurchlässige Unterlage (12); und
einen Absorber (10), der zwischen der Oberlage (11) und der Unterlage (12) angeordnet ist, wobei
ein komprimierter Teil, der ausgebildet ist Kompressionen von der Oberlage (11) zu dem Absorber (10) aufzuweisen, in einer longitudinalen Richtung des absorbierenden Artikels (1) vorgesehen ist,
der komprimierte Teil in wenigstens einen mittleren komprimierten Teil (21) und einen hinteren komprimierten Teil geteilt ist,
der mittlere komprimierte Teil (21) in der longitudinalen Richtung vorgesehen ist, um eine Mittellinie (a-a) eines Ausscheidungsteilkontaktteils des absorbierenden Artikels (1) zu schneiden,
der hintere komprimierte Teil ist dahinter in der longitudinalen Richtung (L) von der Mittellinie (a-a) des Ausscheidungsteilkontaktteils,
ein hinteres Ende (21B) des mittleren komprimierten Teils (21) dahinter in der longitudinalen Richtung (L) von einem vorderen Ende (22F) des hinteren komprimierten Teils vorgesehen ist, und
das hintere Ende (21B) des mittleren komprimierten Teils (21) nach außen in einer Breitenrichtung (W) des absorbierenden Artikels (1) von dem vorderen Ende (22F) des hinteren komprimierten Teils vorgesehen ist, **dadurch gekennzeichnet, dass**
eine Kleidungskontaktoberflächenseite der Unterlage (12) über einen Mittelteil (A), der einem Teil, der über den mittleren komprimierten Teil (21) verfügt, entspricht, verfügt und ein hinterer Teil (B), der dahinter in der longitudinalen Richtung (L) von dem Mittelteil (A) vorgesehen ist,
an dem Mittelteil (A), ein Klebstoff zum Ankleben einer Kleidungskontaktoberfläche der Unterlage (12) an eine Innenoberfläche von Unterwäsche nicht auf einen Teil, der einem Äußeren in der Breitenrichtung (W) des mittleren komprimierten Teils (21) entspricht, aufgetragen wird, jedoch auf einen Teil, der einem Inneren in der Breitenrichtung des mittleren komprimierten Teils (21) entspricht, aufgetragen wird, und
an dem hinteren Teil (B), der Klebstoff nicht auf einen Nachbarteil der Mittellinie (O) in der Breitenrichtung (W) aufgetragen wird, jedoch auf ein Äußeres in der Breitenrichtung (W) des Nachbarteils der Mittellinie (O) in der Breitenrichtung (W) aufgetragen wird.

2. Der absorbierende Artikel gemäß Anspruch 1, wobei der mittlere komprimierte Teil (21) ausgebildet ist, um sich nach außen in der Breitenrichtung (W) zu erstrecken, während er sich in Richtung des hinteren Endes an dem hinteren Teil (B) in der longitudinalen Richtung erstreckt.

3. Der absorbierende Artikel gemäß Anspruch 1 oder 2, wobei der hintere komprimierte Teil ausgebildet ist, um sich nach innen in der Breitenrichtung (W) zu verengen, während er sich in Richtung des vorderen Endes an einem vorderen Teil in der longitudinalen Richtung (L) erstreckt.

4. Der absorbierende Artikel gemäß einem der Ansprüche 1 bis 3, wobei der hintere komprimierte Teil in wenigstens einen ersten hinteren komprimierten Teil (22) und einen zweiten hinteren komprimierten Teil (23) geteilt ist, und
ein hinteres Ende des ersten hinteren komprimierten Teils (22) dahinter in der longitudinalen Richtung und nach innen in der Breitenrichtung hinsichtlich eines vorderen Endes des zweiten hinteren komprimierten Teils (23) vorgesehen ist.

5. Der absorbierende Artikel gemäß einem der Ansprüche 1 bis 4, wobei eine Kleidungskontaktoberflächenseite der Unterlage (12) über ein überlappendes Teil als ein Teil, das sich in der Breitenrichtung von einer Seitenkante zu der anderen Seitenkante des absorbierenden Artikels (1) erstreckt, sowie ein Teil, das zu einem Teil, das das hintere Ende des mittleren komprimierten Teils (21) und das vordere Ende des hinteren komprimierten Teils umfasst, korrespondiert, verfügt und
ein Klebstoff zum Ankleben einer Kleidungskontaktoberfläche der Unterlage (12) an eine innere Oberfläche von Unterwäsche nicht auf den überlappenden Teil aufgetragen wird, jedoch auf einen Teil außer dem überlappenden Teil an der Kleidungskontaktoberflächenseite der Unterlage (12) aufgetragen wird.

6. Der absorbierende Artikel gemäß einem der Ansprüche 1 bis 5, wobei mittlere Flügelteile (40) vorgesehen sind, die zum an beiden Seiten in der Breitenrichtung nach außen Hervorstehen ausgebildet sind, und
die Mittellinie des Ausscheidungskontaktteils zu einer Mittellinie der mittleren Flügelteile (40) in der longitudinalen Richtung korrespondiert.

7. Der absorbierende Artikel gemäß einem der Ansprüche 1 bis 5, wobei
mittlere Flügelteile (40), die zum an beiden Seiten in der Breitenrichtung nach außen Hervorstehen ausgebildet sind, nicht vorgesehen sind und
die Mittellinie des Ausscheidungskontaktteils eine Linie ist, die sich in der Breitenrichtung an einer Position mit der kürzesten Länge in der Breitenrichtung des absorbierenden Artikels (1) erstreckt.

## Revendications

1. Article absorbant (1), comprenant :
une feuille supérieure (11) perméable aux liquides ;
une feuille arrière (12) imperméable aux liquides ; et
un absorbeur (10) disposé entre la feuille supérieure (11) et la feuille arrière (12), dans lequel
une partie comprimée configurée pour permettre une compression de la feuille supérieure (11) vers l'absorbeur (10) est disposée dans une direction longitudinale de l'article absorbant (1),
la partie comprimée est divisée en au moins une partie comprimée centrale (21) et une partie comprimée arrière,
la partie comprimée centrale (21) est disposée dans la direction longitudinale de manière à croiser une ligne centrale (a-a) d'une partie de contact de partie d'excrétion de l'article absorbant (1),
la partie comprimée arrière est disposée postérieurement dans la direction longitudinale (L) à partir de la ligne centrale (a-a) de la partie de contact de partie d'excrétion,
une extrémité arrière (21B) de la partie comprimée centrale (21) est disposée postérieurement dans la direction longitudinale (L) à partir d'une extrémité avant (22F) de la partie comprimée arrière, et
l'extrémité arrière (21B) de la partie comprimée centrale (21) est disposée vers l'extérieur dans une direction de la largeur (W) de l'article absorbant (1) à partir de l'extrémité avant (22F) de la partie comprimée arrière, **caractérisé en ce que**
un côté de surface de contact de vêtement de la feuille arrière (12) est doté d'une partie centrale (A) correspondant à une partie dotée de la partie comprimée centrale (21), et une partie arrière (B) disposée postérieurement dans la direction longitudinale (L) à partir de la partie centrale (A),
au niveau de la partie centrale (A), un adhésif pour l'adhérence d'une surface de contact de vêtement de la feuille arrière (12) à une surface interne de sous-vêtement n'est pas revêtu sur une partie correspondant à un extérieur dans la direction de la largeur (W) de la partie comprimée centrale (21) mais revêtu sur une partie correspondant à un intérieur dans la direction de la largeur de la partie comprimée centrale (21), et
au niveau de la partie arrière (B), l'adhésif n'est pas revêtu sur une partie voisine d'une ligne centrale (O) dans la direction de la largeur (W) mais revêtu sur un extérieur dans la direction de la largeur (W) de la partie voisine de la ligne centrale (O) dans la direction de la largeur (W).

2. Article absorbant selon la revendication 1, dans lequel la partie comprimée centrale (21) est formée de manière à se dilater vers l'extérieur dans la direction de la largeur (W) lorsqu'elle s'étend en direction de l'extrémité arrière au niveau de la partie arrière (B) dans la direction longitudinale.

3. Article absorbant selon la revendication 1 ou 2, dans lequel la partie comprimée arrière est formée de manière à se rétrécir vers l'intérieur dans la direction de la largeur (W) lorsqu'elle s'étend en direction de l'extrémité avant au niveau d'une partie avant dans la direction longitudinale (L).

4. Article absorbant selon l'une quelconque des revendications 1 à 3, dans lequel
la partie comprimée arrière est divisée en au moins une première partie comprimée arrière (22) et une seconde partie comprimée arrière (23), et
une extrémité arrière de la première partie comprimée arrière (22) est disposée postérieurement dans la direction longitudinale et vers l'intérieur dans la direction de la largeur par rapport à une extrémité avant de la seconde partie comprimée arrière (23).

5. Article absorbant selon l'une quelconque des revendications 1 à 4, dans lequel
un côté de surface de contact de vêtement de la feuille arrière (12) est doté d'une partie de chevauchement comme une partie s'étendant dans la direction de la largeur d'un bord latéral vers l'autre bord latéral de l'article absorbant (1) ainsi qu'une partie correspondant à une partie comprenant l'extrémité arrière de la partie comprimée centrale (21) et l'extrémité avant de la partie comprimée arrière, et
un adhésif pour l'adhérence d'une surface de contact de vêtement de la feuille arrière (12) à une surface interne de sous-vêtement n'est pas revêtu sur la partie de chevauchement mais revêtu sur une partie autre que la partie de chevauchement au niveau du côté de surface de contact de vêtement de la feuille arrière (12).

6. Article absorbant selon l'une quelconque des revendications 1 à 5, dans lequel
des parties d'aile centrales (40) configurées pour faire saillie vers l'extérieur au niveau des deux côtés dans la direction de la largeur sont fournies, et
la ligne centrale de la partie de contact d'excrétion correspond à une ligne centrale des parties d'aile centrales (40) dans la direction longitudinale.

7. Article absorbant selon l'une quelconque des revendications 1 à 5, dans lequel
des parties d'aile centrales (40) configurées pour faire saillie vers l'extérieur au niveau des deux côtés dans la direction de la largeur ne sont pas fournies, et
la ligne centrale de la partie de contact d'excrétion est une ligne qui s'étend dans la direction de la largeur au niveau d'une position présentant la longueur la plus courte dans la direction de la largeur de l'article absorbant (1).
